# EUROPEAN PATENT APPLICATION

(11) **EP 3 912 991 A1**
(43) Date of publication of application: **24.11.2021**
(21) Application number: 20315243.4
(22) Date of filing: 18.05.2020
(51) Int. Cl.: C07K 14/47, A61K 38/17

(54) **THERAPEUTIC POTENTIAL OF PEPTIDES FROM THE "NETRIN-LIKE" DOMAIN OF THE FRZB PROTEIN**

(71) Applicant: Université de Lorraine, 54000 Nancy (FR); Centre National de la Recherche Scientifique, 75016 Paris (FR)
(72) Inventor: Cailotto, Frédéric, 54520 Laxou (FR); Claudel, Marion, 54000 Nancy (FR); Charron, Christophe, 54110 Flainval (FR); Jouzeau, Jean-Yves, 54230 Chaligny (FR)
(74) Representative: Novagraaf Technologies

(57) **Abstract**

The present invention concerns new peptides derived from the netrin-like domain of the FRZB protein, that are useful in the prevention or treatment of Wnt/β-catenin and/or Hippo/YAP/TAZ pathway-related diseases, in particular of osteoarthritis.

## Description

### Technical field of the invention

The present invention concerns new peptides derived from the netrin-like domain of the FRZB protein, that are useful in the prevention or treatment of Wnt/β-catenin and/or Hippo/YAP/TAZ pathway-related diseases, in particular of osteoarthritis.

In the description below, references in square brackets ([ ]) refer to the list of references at the end of the text.

### State of the art

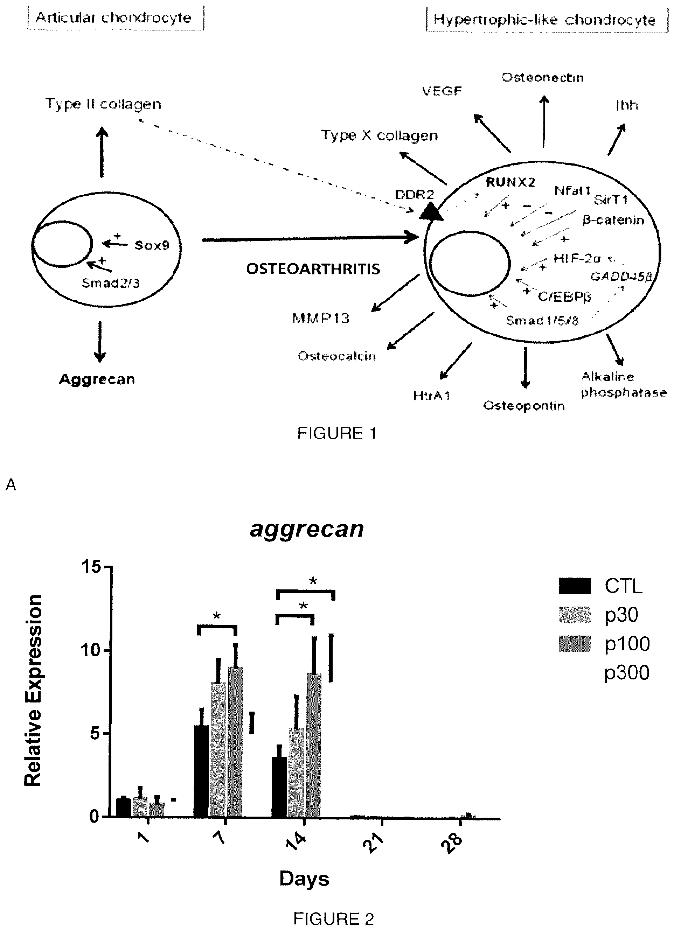

The synovial joints are specialized structures that connect the bones and support movement while at the same time restricting the range of motion between the different skeletal elements. In these specialized organ-like structures, the bony bearings are capped with a thin layer of hyaline or articular cartilage. The joint cavity in between is lined by the synovial membrane. Together, these different tissues allow smooth movement with very low-friction and sufficient lubrication between joint surfaces. Homeostasis of the joint, in particular of the articular cartilage and subchondral bone is essential for maintenance of joint function and critically dependent on the balance between anabolic and catabolic signaling pathways (Lories and Luyten, 2011) [1]. The homeostasis requires maintenance of the stable chondrocyte phenotype that characterizes the articular cartilage, controlled by soluble factors and extracellular matrix (ECM), under the form of either whole or cleaved proteins. Major phenotypic changes in the course of OA are depicted in Figure 1.

Loss of homeostasis results in gradual deterioration of cartilage quality and thickening of the subchondral bone, and low to medium synovial inflammation, leading to osteoarthritis (OA). The OA diseases are a group of disorders characterized by joint pain and loss of function in the absence of chronic autoimmune mechanisms. The prevalence of OA is increasing with age and represents an enormous socio-economic challenge with millions of people affected worldwide (Hunter and Felson, 2006) [2]. Aging of the population and epidemic increases in some of the risk factors such as obesity suggest that OA associated problems and disability will only increase their impact on society. Drug therapy options today are still very limited, including painkillers and non-steroidal anti-inflammatory drugs. In advanced OA joint prosthesis surgery is often required (Hunter and Felson, 2006) [2].

Epidemiological and clinical data support the view that OA is a disease with a complex aetiology to which both genetic and acquired or environmental factors contribute (Valdes and Spector, 2008) [3]. These factors interact and determine the onset, progression and outcome of the disease. The identification of genetic factors that are associated with OA also conveys important information about pathways that are important for skeletal development or for maintenance of homeostasis in the adult joint tissues and identifies therapeutic targets to maintain joint homeostasis. Polymorphisms in susceptibility genes are associated with OA without causing overt skeletal abnormalities. These associations include polymorphisms in genes known from their roles in skeletal development such as secreted frizzled-related protein (SFRP) 3, initially named FRZB, a natural Wnt modulator. FRZB contains two remarkable domains, the cysteine-rich domain (CRD_{FRZB}), involved in interacting with Wnt ligands, and the Netrin-like domain (NTN_{FRZB}). Work from Pr. Lories' laboratory has demonstrated that FRZB deficiencies lead to OA in different mice models (Lories et al., 2007) [4]. Then, it was showed in this laboratory that mice deficient for FRZB underwent a significant modification of their transcriptome, with an increase in the activity of the canonical Wnt pathway (Lodewyckx et al., 2012) [5]. Moreover, an increase in the concentration of Dickopf-related protein-1 (Dkk1, a Wnt antagonist) has been associated with a decreased risk of joint space narrowing, a radiographic sign validated as a marker of articular cartilage loss in human OA (Lane et al., 2007) [6].

Furthermore, the canonical Wnt pathway, involving β-catenin activation, contributes to the control of cartilage homeostasis. On one hand, some studies demonstrated that β-catenin could preserve the integrity of articular cartilage. For example, a very recent study demonstrated that activation of β-catenin prevented the hedgehog-induced cartilage degradation in mature articular cartilage (Rockel et al., 2016) [7]. Moreover, the group from Di Chen showed that transgenic mice expressing the inhibitor of β-catenin and T-cell factor (the transcription factor regulating the Wnt canonical pathway) specifically in articular cartilage (under the control of Col2a1 promoter), developed significant cartilage destruction compared to controls (Zhu et al., 2008) [8]. On the other hand, several studies put in light the deleterious influence of β-catenin activation whose excess leads to cartilage degradation (Tamamura et al., 2005) [9]. Moreover, constitutive activation of β-catenin in articular chondrocytes from adult mice (knock-in for constitutively active β-catenin under the control of Col2a1 promoter), also from the group of Di Chen, resulted in OA-like features, including high MMP-13, type X collagen and osteocalcin expression (Zhu et al., 2009) [10].

Another signaling pathway, the Hippo/YAP/TAZ pathway, is also linked to the pathophysiology of OA (Gong et al., 2019) [11]. In this work, blocking the YAP pathway using siRNA (targeting YAP) allowed to reduce the progression of OA *in vivo* in a mouse model of the pathology. Very recently, the use of Verteporfin, a YAP inhibitor, allowed preserving the cartilage homeostasis in a mouse model of OA (Zhang et al., 2020) [12].

While activation of Calmodulin-Kinase II (Camkll) was shown to trigger hypertrophy in growth plate chondrocytes (Li et al., 2011) [13], this effect required a simultaneous activation of β-catenin. Moreover, it was shown at the world congress on osteoarthritis in 2016 that selective antagonism of Camkll activation worsened the onset and progression of OA in a mouse model (Nalesso et al., 2016) [14]. Finally, it was demonstrated recently that the truncated form of FRZB, containing only the Netrin-like domain (NTN_{FRZB}) was able to strongly activate the non-canonical Wnt pathway (through activation of the CamKII), while inhibiting the canonical pathway. This was shown in an osteogenesis (Thysen et al., 2014) [15] and in a chondrogenesis model (Thysen et al., 2014) [16], both tightly controlled by Wnt signals, resulting in that case in an increased early chondrogenesis process (type II collagen and aggrecan increase).

Very interestingly, both Wnt/β-catenin (Cui et al., 2018) [17] and Hippo/YAP/TAZ (Zheng et al., 2019) [18] pathways were extensively described as important contributors to cancer onset and/or development. Therefore, inhibitors of such pathways could represent potentially interesting leads in the field of cancer treatment.

The size of the whole protein FRZB, which has already been shown to be effective in modulating the signalling pathways involved in osteoarthritis (as well as its truncated form containing only the netrin-like domain), is 34 kDa. Because of its large size, this protein or its truncated form may be immunogenic in *in vivo* experiments. This is why it may be interesting to derive peptides from the protein FRZB having the same or similar properties (biological activity) than the whole protein in order to reduce the risk of an immune system reaction.

### Description of the invention

The inventors have unexpectedly identified new peptides derived from the netrin-like domain of the FRZB protein having the ability to decrease the expression of the Wnt/β-catenin canonical pathway that is overexpressed in osteoarthritis, decrease the expression of the Hippo/YAP/TAZ pathway involved in the pathophysiology of osteoarthritis, and increase the expression of the non-canonical CamKII pathway having a protective role for the joint.

The peptides were originally designed with the idea that in the sequence of the netrin-like domain of the FRZB protein, there may be elements suggesting an alpha-helix structuring, allowing the interaction of said domain with other protein partners. Therefore, two peptide sequences PEP12 (ATQKTYFRNNYN, SEQ ID NO: 4) and PEP16 (DRLGKKVKRWDMKLRH, SEQ ID NO: 2) derived from the netrin-like domain and corresponding to this criterion, have been tested. However, it turned out that PEP12, although chosen by computer prediction to have a similar secondary, alpha-helix conformation, to that of PEP16, was totally ineffective in the tested systems described below, unlike PEP16. It then appeared that deriving active peptides from the netrin-like domain of the FRZB protein was not as obvious as expected.

PEP16 is a peptide which sequence is predicted to have a propensity to form an alpha-helix, and which has a role in the regulation of the canonical (Wnt/β-catenin) and non-canonical (CamKII) Wnt pathways: it decreases activation of the canonical pathway which is over-expressed in osteoarthritis and increases activation of the non-canonical pathway having a protective role for the joint. In addition, PEP16 is capable of reducing the activation of the Hippo/YAP/TAZ pathway notably involved in the physiopathology of osteoarthritis. In order to specifically target osteoarthritis cartilage, PEP16 can be vectorised with an addressing sequence specific for binding to collagen 2a1 (col2a1).

Considering that the alpha-helix conformation was essential to the activiy of PEP16, the inventors then derived mutated sequences from the PEP16 peptide to promote this alpha-helix conformation. The inventors have also included among the five sequences tested, a mutant sequence without the tryptophan residue that could be strongly linked to the activity of PEP16 (binding). This mutant sequence might then constitute a negative control of the activity, as the steric hindrance of this sequence could constitute a more selective binding via the tryptophan residue. In these mutant sequences, the mutations of the hydrophobic amino acids into alanine made it possible to respect the predicted helicoidal shape, while favouring the turns of the helix because alanine has this structural property, therefore potentially increasing the rigidity of the helix. Moreover, the inversions of lysine to arginine allowed to preserve the notion of electric charge of the peptide, and its total global charge, while altering its primary sequence, to elucidate a possible effect which would be solely due to the electric charge. The five mutant sequences are as follows (in bold the variations from PEP16):
DR**A**GKKVKRWDMK**A**RH (SEQ ID NO: 3)
DRLGKKVK**A**WDMKLRH (SEQ ID NO: 5)
D**K**LG**RR**V**RK**WDM**RLK**H (SEQ ID NO: 6)
DR**A**GKK**A**KRWDMK**A**RH (SEQ ID NO: 7)
DRLGKKVKR**A**DMKLRH (SEQ ID NO: 8)

One of which (PEP16bis: DRAGKKVKRWDMKARH, SEQ ID NO: 3) was particularly interesting since it had very similar properties (biological activity) to the initial sequence PEP16 in the reporter system of the Super TOPflash canonical Wnt pathway.

Therefore an object of the present invention concerns a peptide of 16 to 50 amino acids comprising a peptide having the following sequence :
DRX₃GKKVKRWDMKX₁₄RH (SEQ ID NO: 1)
wherein X₃ and X₁₄ are independently of each other any amino acid.

According to a particular embodiment of the peptide of the present invention, X₃ and X₁₄ are independently of each other an alanine or a leucine. For example, the peptide of the present invention comprises or consists of the sequence SEQ ID NO: 2 (DRLGKKVKRWDMKLRH) or the sequence SEQ ID NO: 3 (DRAGKKVKRWDMKARH) .

Another object of the present invention concerns a peptide of the present invention for use as a drug.

Another object of the present invention concerns a pharmaceutical composition comprising a peptide of the present invention, and one or more pharmaceutically acceptable excipient.

Another object of the present invention concerns a peptide or pharmaceutical composition of the present invention for use in the prevention or treatment of a Wnt/β-catenin and/or Hippo/YAP/TAZ pathway-related disease selected from the group consisting of: osteoarthritis, osteoporosis and cancer. Preferably, it concerns a peptide or pharmaceutical composition of the present invention for use in the prevention or treatment of osteoarthritis.

### Brief description of the figures

Figure 1 represents alterations of the articular chnodrocyte's phenotype in the course of OA. The phenotype biomarkers are indicated around the cells, and the main modulators of phenotype are indicated inside the cells. + : stimulating effect, - : inhibiting effect.

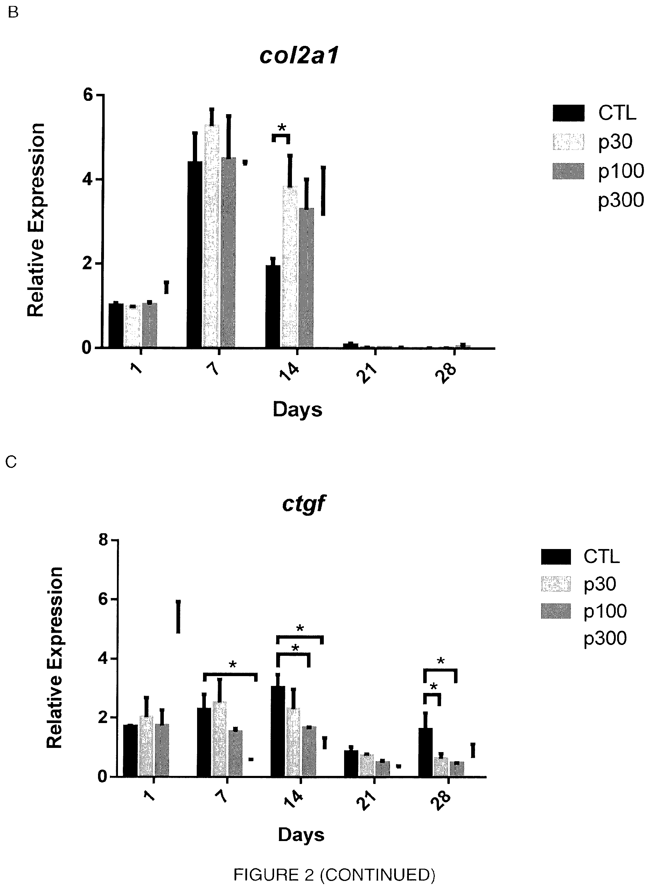

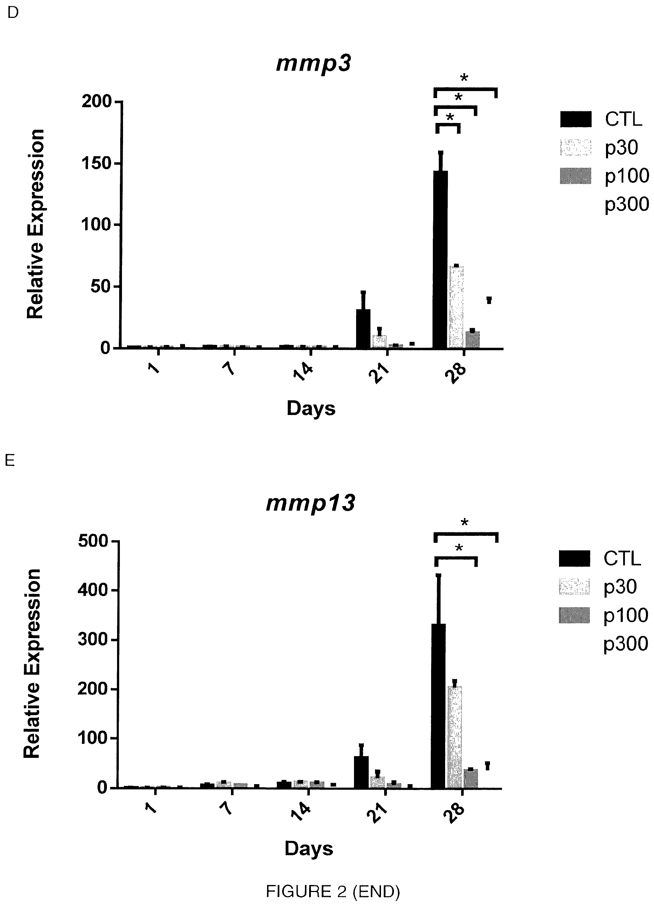

Figure 2 represents the relative expression level of (A) *aggrecan,* (B) *col2a1,* (C) *ctgf,* (D) *mmp3* and (E) *mmp13,* at day 1, 7, 14, 21 and 28 in ATDC5 micromasses, treated with the PEP16 at 30, 100, 300 ng/mL (respectively, P30, P100 and P300), compared to a non-treated control (CTL). Results were normalized with the housekeeping gene *ppia.* *P< 0.05. Results are representative of 3 experiments.

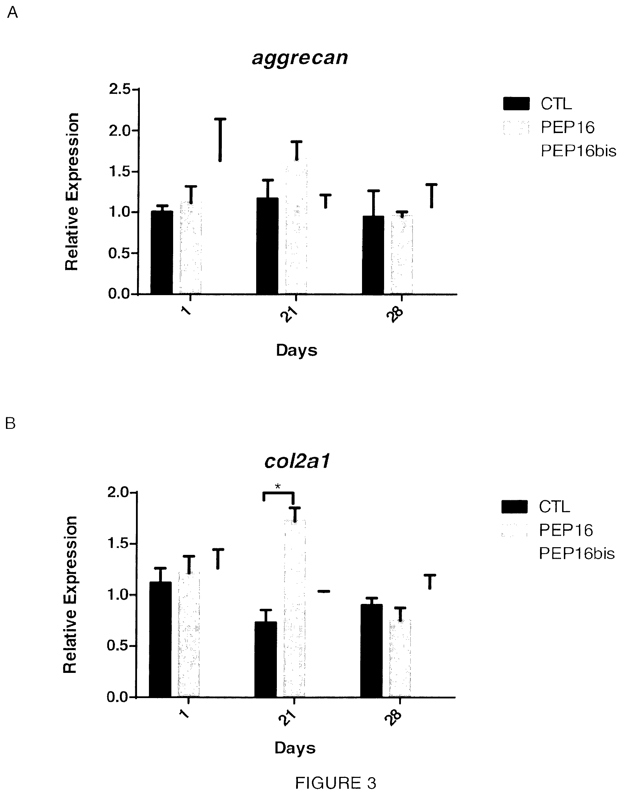

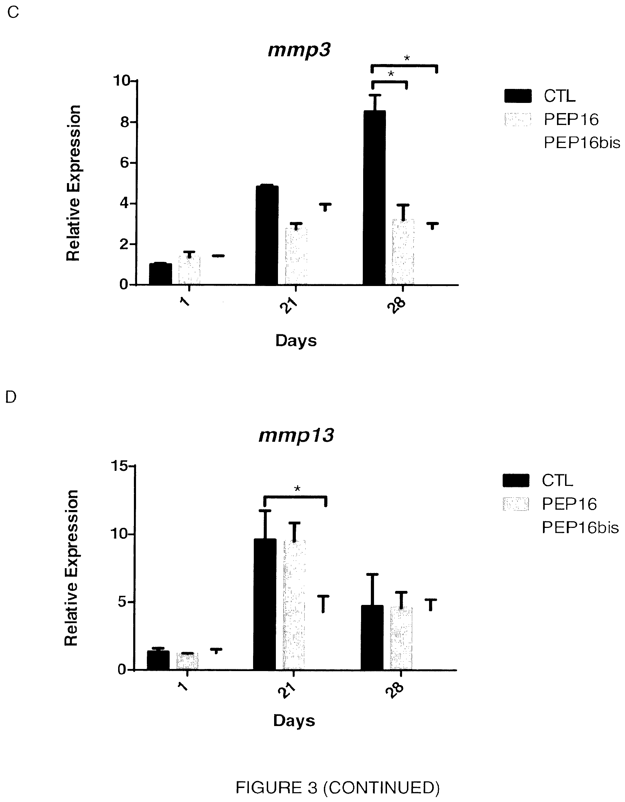

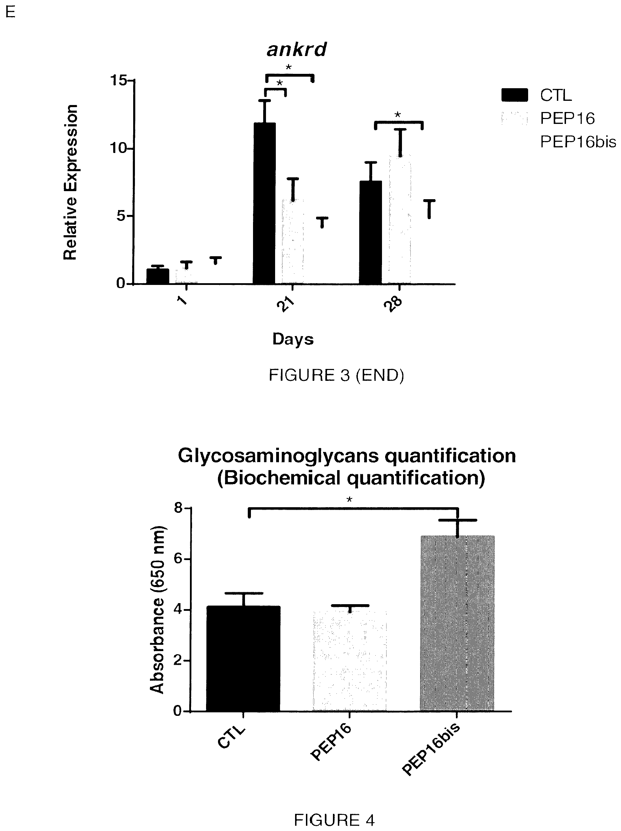

Figure 3 represents the relative expression level of (A) *aggrecan,* (B) *col2a1,* (C) *mmp3,* (D) *mmp13,* and (E) *ankrd* at day 1, 21 and 28 in ATDC5 micromasses treated with the PEP16 and PEP16bis at 300 ng/mL, compared to a non-treated control (CTL). Results were normalized with the housekeeping gene *ppia.* *P< 0.05. Results are representative of 2 experiments (3 experiments in (E)).
Figure 4 represents glycosaminoglycans quantification in ATDC5 micromasses treated with the PEP16 and the PEP16bis at 300 ng/mL and compared to a non-treated control (CTL). Alcian blue staining has been performed at day 28. Results are expressed in absorbance (650nm) after a biochemical quantification. *P<0.05. Results are representative of 2 experiments.

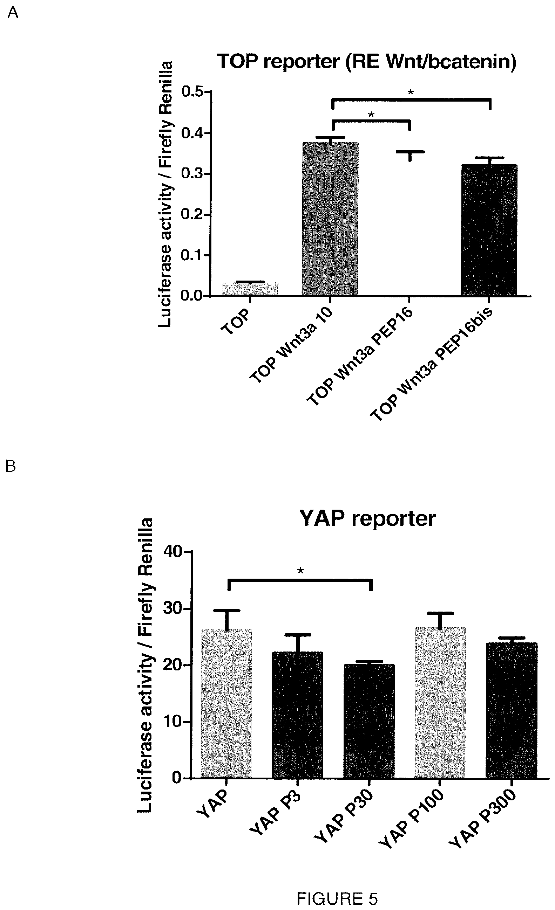

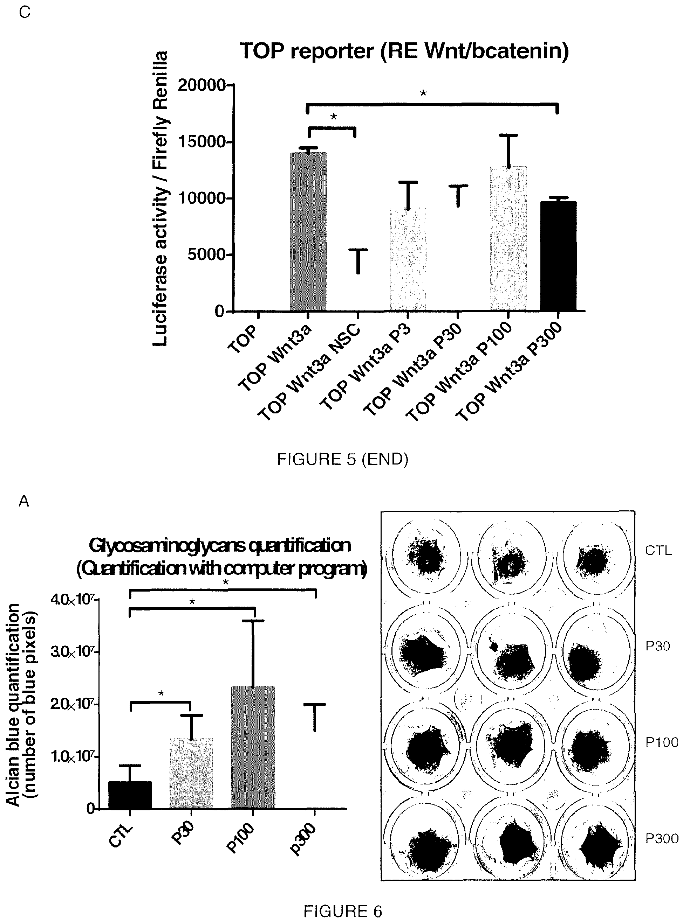

Figure 5 represents transcriptionnal activity of promoters of interest evaluated using constructs containing the firefly luciferase gene under the control of (A) 7X TCF/LEF response elements (Super 8x TOPFlash reporter) in ATDC5 micromasses treated one day after transfection with the PEP16 or the PEP16bis at 300 ng/mL, and simultaneously the Wnt3a at 100 ng/mL, for 24h, (B) 8X YAP reponse elements (8x GTIIC reporter) or (C) 7X TCF/LEF response elements (Super 8x TOPFlash reporter) in ATDC5 micromasses treated one day after transfection with the PEP16 at 3, 30, 100, 300 ng/mL (respectively P3, P30, P100 and P300), and simultaneously with the Wnt3a at 100 ng/mL, for 24 h. Data were normalized using the Renilla luciferase under the control of tyrosine kinase promoter (TK-RL). Results are expressed as the ratio of Firefly /renilla luciferase activity. The NSC668036 (NSC, broad inhibitor of Wnt/β-catenin signaling) was used as a positive control for Wnt3a antagonism. *P<0.05. Results are representative of 2 experiments.

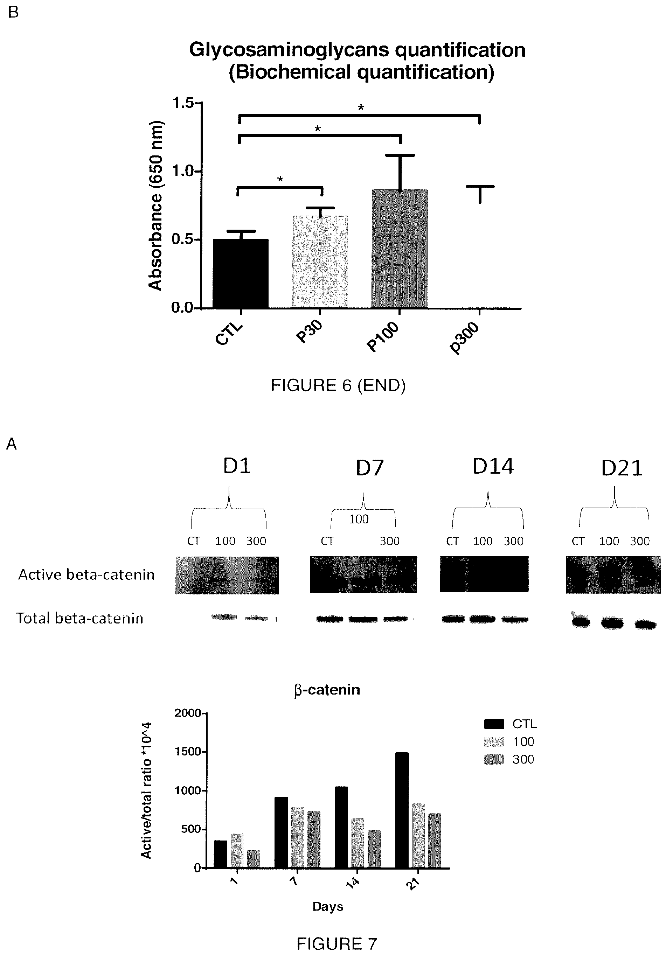

Figure 6 represents glycosaminoglycans quantification (A) with computer program (blue pixels quantification) (B) with biochemical quantification (solubilisation of the staining in Guanidine HCI 6M), in ATDC5 micromasses treated with the PEP16 at 30, 100 and 300 ng/mL (respectively, P30, P100 and P300) and cultured during 28 days in comparison of a non-treated control (CTL). Alcian blue staining was performed at day 28. *P<0.05. Results are representative of 3 experiments.

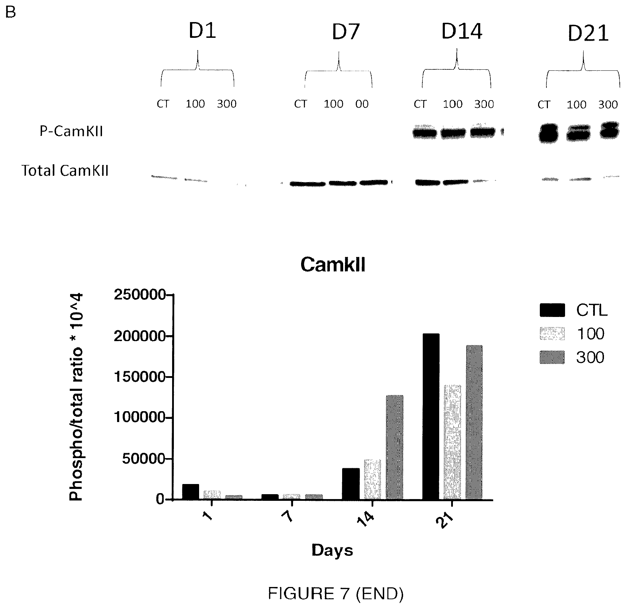

Figure 7 represents western blot exploring (A) the Wnt/β-catenin pathway activation (B) the CamkII pathway activation, in ATDC5 micromasses at day 1, 7, 14, and 21 after treatment with the PEP16 at 100 and 300 ng/mL in comparison to a non-treated control (CT or CTL). Results are expressed in active protein form over total protein form. Results are representative of 3 experiments.

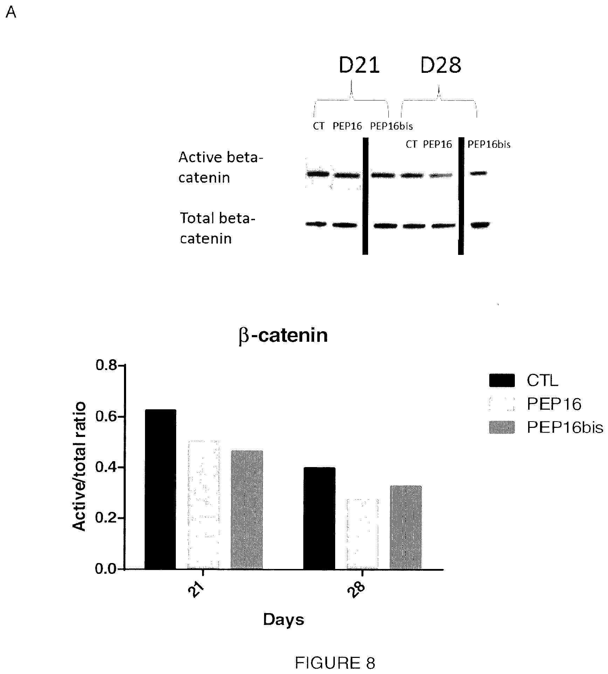

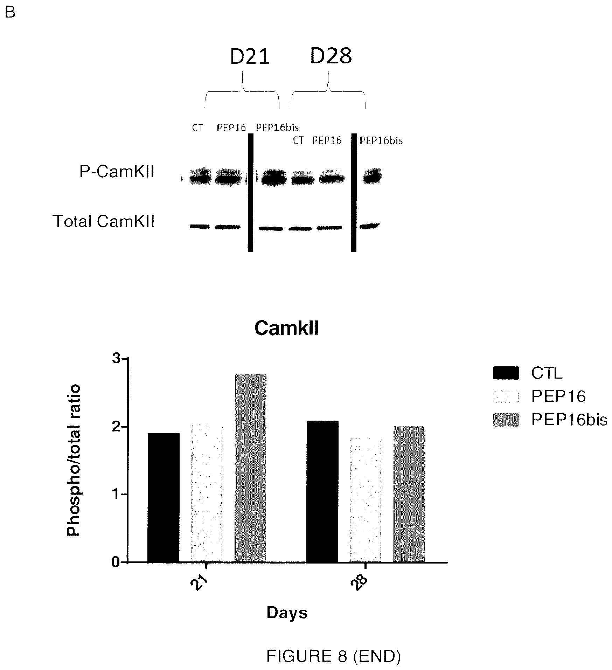

Figure 8 represents western blot exploring (A) the Wnt/β-catenin pathway activation (B) the CamKII pathway activation, in ATDC5 micromasses at day 21 and 28 after treatment with the PEP16 or the PEP16bis at 300 ng/mL in comparison to a non-treated control (CT or CTL). Results are expressed in active protein form (*i.e.* P-CamKII for (B)) over total protein form. Results are representative of 3 experiments.

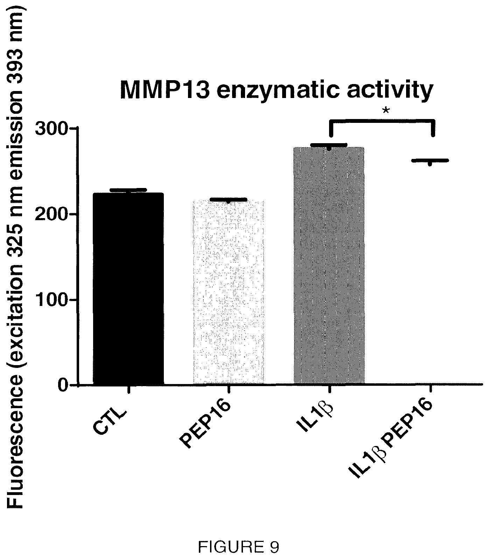

Figure 9 represents MMP13 enzymatic activity in culture supernatants of mouse articular chondrocytes treated during 24h with PEP16 at 300 ng/mL and/or with IL1β at 100 pg/mL and compared to a non-treated control (CTL). IL1β was used as a positive control for MMP13 activity. *P<0.05.

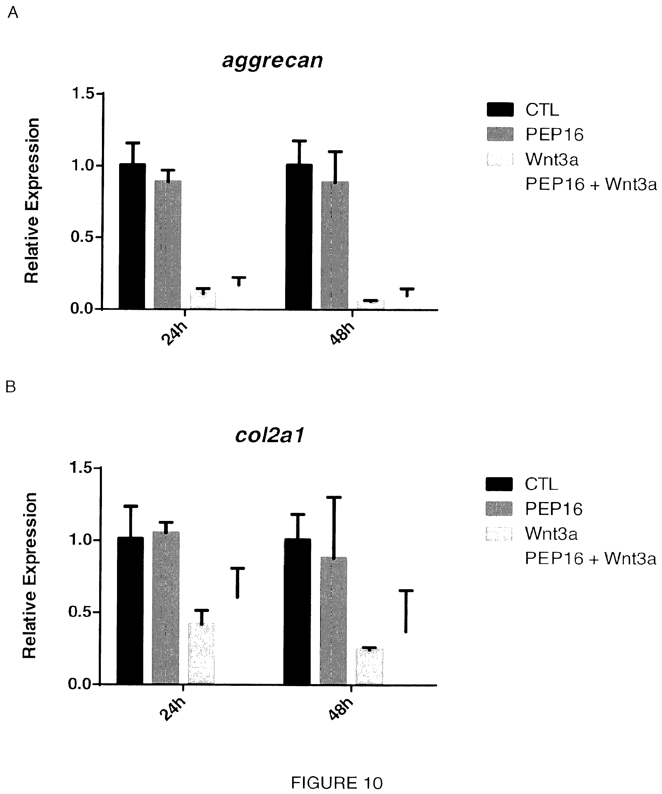

Figure 10 represents the relative expression level of (A) *aggrecan,* (B) *col2a1,* at 24 and 48h in mouse articular chondrocytes treated with the PEP16 at 300 ng/mL and/or wnt3a at 100 ng/mL, compared to a non-treated control (CTL). Results were normalized with the housekeeping gene *ppia.* Results are representative of 3 experiments.

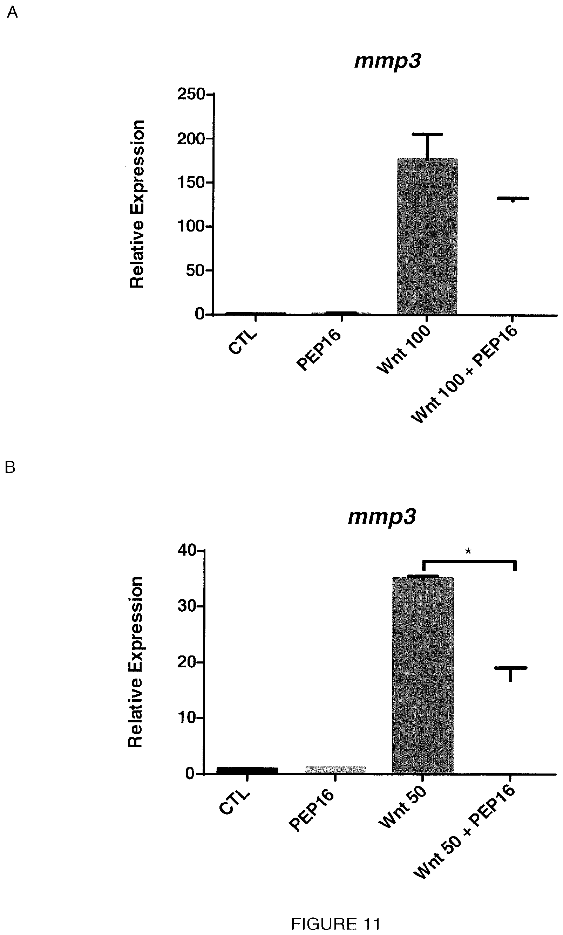

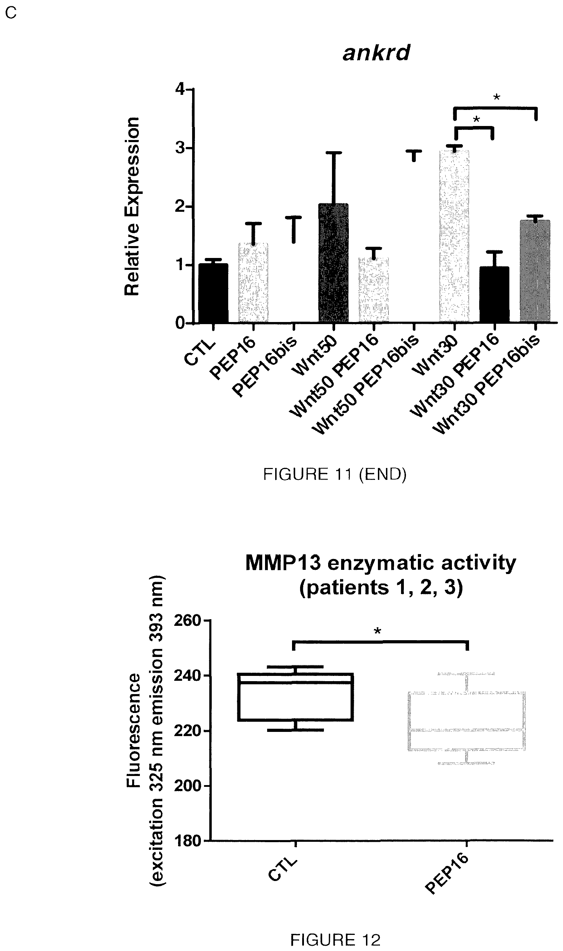

Figure 11 represents the relative expression level of (A, B) *mmp3* and (C) *ankrd,* in mouse articular chondrocytes treated with the PEP16 or PEP16bis at 300 ng/mL, and/or wnt3a at 100, 50, 30 ng/mL, compared to a non-treated control (CTL). Results were normalized with the housekeeping gene *ppia.* *P< 0.05. Mmp3 expression was monitored after 24h of stimulation, while ankrd expression was monitored after 12h of stimulation. Results are representative of 3 experiments.
Figure 12 represents the MMP13 enzymatic activity in culture supernatants of human articular cartilage explants (from 3 patients with end-stage osteoarthritis following total knee replacement surgery) treated with PEP16 at 300 ng/mL for 7 days (3 stimulations in 7 days) and compared to a non-treated control (CTL). *P<0.05. (N=3 patients)

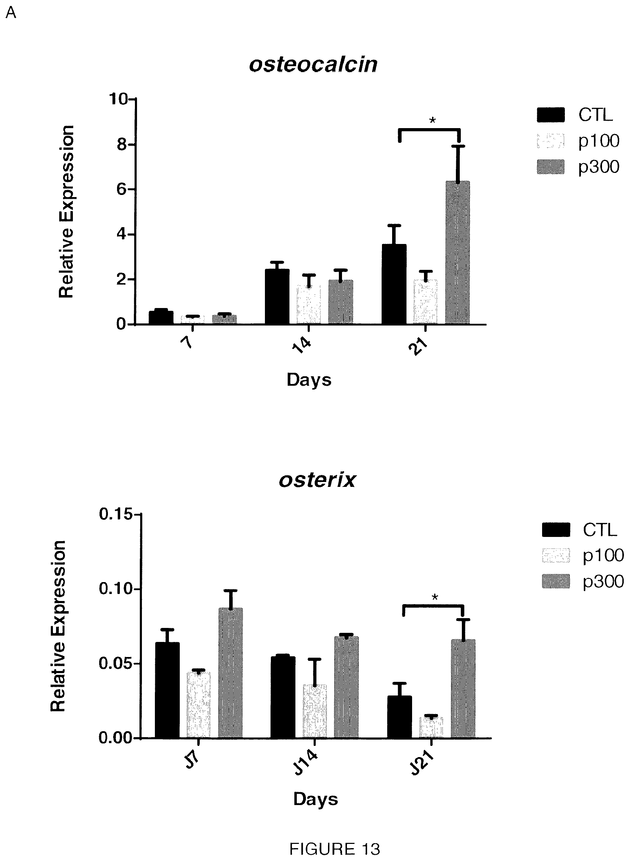

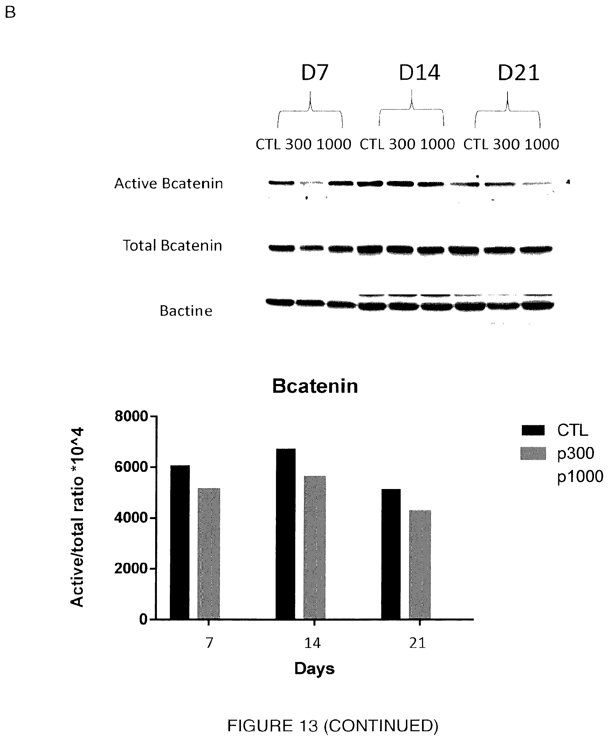

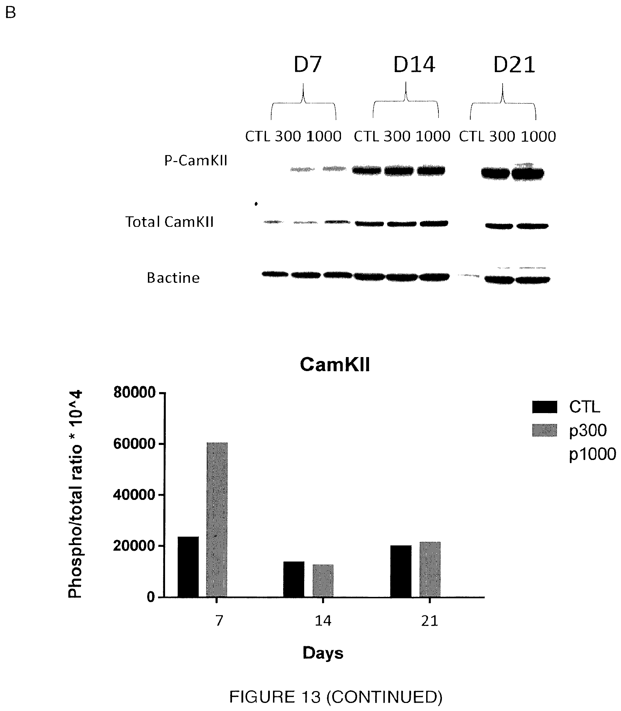

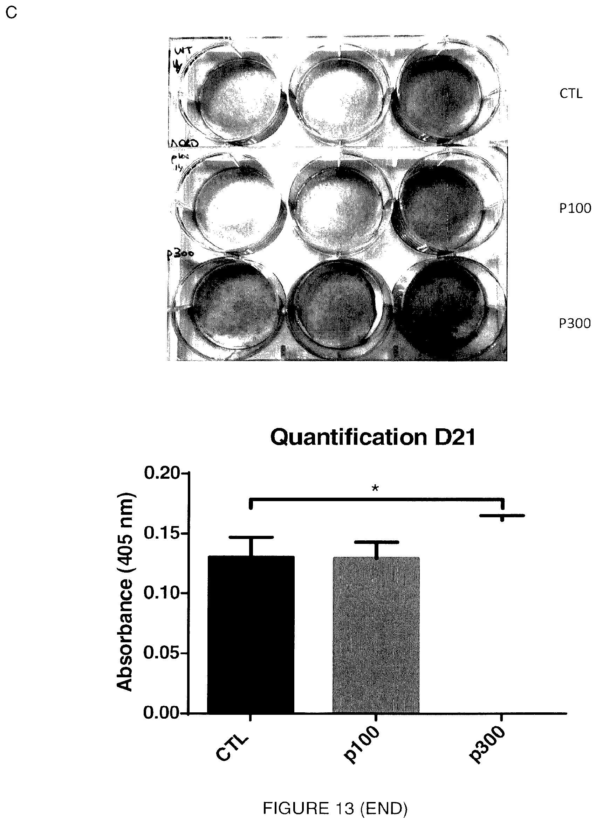

Figure 13 represents the influence of PEP16 on osteogenic differentiation. MC3T3-E1 cells were stimulated with PEP16 at different concentrations for 21 days, and compared to non-treated controls. Results were normalized with the housekeeping gene S29. *P< 0.05. The relative expression level of (A) osteocalcin and osterix was studied, as well as (B) western blot exploring the Wnt/β-catenin pathway and the CamKII pathway activation, and (C) the mineralization level after 21 days of differentiation culture. Results are representative of 3 experiments.

### EXAMPLES

### EXAMPLE 1 : MATERIAL & METHODS

### ATDC5 micromass culture

Mouse chondrogenic ATDC5 cells (cartilage-specific chondrogenic strain) were cultured until confluent in maintenance medium containing 1:1 Dulbecco's modified Eagle's medium (DMEM): Ham's F-12 mix (Gibco) supplemented with 1% antibiotic (AB, Penicillin-Streptomycin, Gibco), 5% fetal bovine serum (FBS) (Gibco) and enriched with 10 µg/ml human transferrin (Sigma) and 30 nM sodium selenite (Sigma). Cells were trypsinized, washed and resuspended at 2.10⁷ cells/mL in a chondrogenic medium made of maintenance medium enriched by 1X of ITS premix (resulting in 10 µg/mL insulin, 10 µg/mL human transferrin and 30 nM sodium selenite) (Life technologies). One droplet of cell suspension (10 µL) was placed at the center of each well of a 24-well plate. Cells were allowed to adhere for 2 h at 37°C, followed by addition of 500 µL chondrogenic medium supplemented. During the whole culture, the cells were cultured in the absence or in the presence of PEP16 (SEQ ID NO: 2) and/or PEP16bis (SEQ ID NO: 3) at different concentrations (30 ng/mL, 100 ng/mL and/or 300 ng/mL). After 14 days, induction of hypertrophic differentiation and mineralization was induced by the mineralization medium made of α-MEM medium (Gibco) containing 1% AB, 5% FBS, 5 µg/ml human transferrin, 1X of ITS premix, 50 µg/ml ascorbic acid-2-phosphate (AA) (Sigma) and 10 mM β-glycerophosphate (BGP) (Sigma). Cells were maintained in a humidified atmosphere of 5% CO2 at 37°C. Micromasses were collected at time points 1, 7, 14 21 and 28 days. Each time point was processed in three technical replicates. The medium was changed every other day. Readouts included proteoglycans content, signaling pathways activation status and genes of interest evaluation. The genes of interest were articular chondrocyte markers (aggrecan, type 2 collagen (col2a1)), YAP/TAZ target genes (ctgf, ankrd) and pro-catabolic enzymes involved in the pathophysiology of OA (mmp3, mmp13).

### MC3T3-E1 monolayer culture

Mouse osteogenic MC3T3-E1 cells (bone-specific osteogenic strain) were cultured until confluent in maintenance medium containing Dulbecco's modified Eagle's medium (DMEM) (Gibco) supplemented with 1% AB (Gibco), 10% FBS (Gibco) and enriched with 1% sodium pyruvate (Gibco). Cells were trypsinized, washed and then seeded at 2600 cells/cm² in 6-well plates. The induction of MC3T3-E1 differentiation was initiated the day after (D1) by culturing cells for 21 days in α-Minimal Essential medium (a-MEM, Gibco), supplemented with 10% FBS, 1% AB, 1% sodium pyruvate, 10 mM BGP and 50 µg/ml AA.

During the whole culture, the cells were cultured in the absence or in the presence of PEP16 (SEQ ID NO: 2) at different concentrations (100 ng/mL, 300 ng/mL or 1000 ng/ml). Cells were maintained in a humidified atmosphere of 5% CO2 at 37°C. Cells were collected at time points 1, 7, 14 21 days. Each time point was processed in three technical replicates. The medium was changed every two days. Readouts included mineral content, signaling pathways activation status and genes of interest evaluation. The genes of interest were osteognic differentiation markers (osteocalcin, osterix).

### Mouse articular chondrocytes culture

Mouse articular chondrocyte (MACs) were isolated from femoral heads by enzymatic digestion of whole femoral head caps. Samples were washed three times in 1% AB/phosphate buffered saline (PBS) and incubated with 2 mg/ml pronase (Roche)/DMEM-F12 at 37°C for 1 h at slow rotation 100 rpm followed by a 3 h incubation with 1,5 mg/ml collagenase B (Roche)/ DMEM-F12 at 37°C. Cells were seeded at 1 x 10⁶ cells / 75 mm², and cultured for 7 to 14 days in growth medium DMEM-F12, containing 1% AB (Gibco), 10% FBS (Gibco) and 5 % L-glutamine (Thermoscientific). Passage 1 cells were utilized for the experiments. The culture supernatants were then harvested in order to perform MMP13 enzymatic activity assay. The evaluation of the expression of genes of interest included articular chondrocyte markers (aggrecan, type 2 collagen (col2a1)), YAP/TAZ target genes (ankrd) and pro-catabolic enzyme involved in the pathophysiology of OA (mmp3).

### Explants of diseased human articular cartilage culture

Explants of human articular cartilage were obtained from total knee replacement surgeries, from the orthopedic department from the CHRU of Nancy (Pr. Didier Mainard). Samples were washed three times in 1% AB/PBS and incubated for 24 h in growth medium (DMEM-F12, containing 1% AB (Gibco), 10% FBS (Gibco) and 5 % L-glutamine (Thermoscientific). Explants were then stimulated with PEP16 at the concentration of 300 ng/ml during 7 days. The culture supernatants were then harvested in order to perform MMP13 enzymatic activity assay.

### Promoter activation assays

ATDC5 cells were transfected with either the YAP/TAZ firefly luciferase reporter plasmid (8x GTIIC, Plasmid #34615, Addgene), the β-catenin payhway reporter plasmid (Super 8x TOPFlash, Plasmid #12456, Addgene) or the inactive β-catenin control reporter plasmid (Super 8x TOPFlash, #12457, Addgene). Results were normalized by-cotransfecting each of the above plasmid with the tyrosine kinase-renilla luciferase plasmid (Promega). Transfections were carried out using the Dharmafect kb transfection reagent (Fisher Scientific). Briefly, reporter plasmids and transfection reagent were diluted in serum-free medium, and then the diluted Dharmafect kb was added to the plasmids for an incubation time of 10 minutes at room temperature. During that time, cells were rinsed with PBS and were placed in fresh maintenance medium. When incubation was over, the mixture of transfection reagent-plasmids was dispensed in each well. Stimulations (with Wnt3a and/or PEP16 and/or PEP16bis) were carried out the next day.

### RNA extraction, cDNA synthesis and Quantitative Real-Time Polymerase Chain Reaction (qRT-PCR) (to see expression of healthy and hypertrophic chondrocyte markers)

Total RNA was isolated using the Nucleospin RNA II kit (Macherey-Nagel) and reverse-transcribed using the MMLV reverse transcriptase (Thermo Fisher Scientific) following the manufacturers' instructions. The iTaq SYBR green qPCR master mix (BioRad) was used to assess the mRNA expression of target genes of interest. Primer sequences can be provided upon request. The following PCR conditions were used: 1 minute at 95°C, 40 cycles of 15 sec of denaturation at 95°C, followed by 1 minute of annealing-elongation at 60°C. Melting curve analysis was performed to determine the amplification of the specific product. Results were expressed using the comparative threshold method and were normalized to housekeeping gene PPIA (Peptidylprolyl Isomerase A) mRNA level for ATDC5 cells and MACs, or normalized to housekeeping gene S29 (ribosomal protein S29) mRNA level for MC3T3-E1 cells. The Via7 real-time PCR system (Thermo Fisher Scientific) was used for qRT-PCR measurements.

### Quantification of proteoglycan formation

Micromasses were washed with PBS and fixed with 95% ice-cold ethanol for 30 min at 4°C for staining. After washing with distilled water, micromasses were stained with Alcian Blue (0.1 % Alcian Blue 8GX, (Sigma)), washed three times with distilled water to remove unbound staining and air-dried. Quantification of the staining was performed by blue pixel quantitation, using the ImageJ software (NIH Image, National Institutes of Health, Bethesda, MD, USA), and then by dissolving the micromasses with 6M Guanidine-HCI (Sigma) and by measuring the absorbance at 650 nm with a spectrophotometer (Varioskan Flash, Thermo Fisher Scientific).

### Quantification of mineralization formation

Cells were washed with PBS and fixed with 95% ice-cold ethanol for 30 min at 4°C for staining. After washing with distilled water, cells were stained with Alizarin Red staining (1 % Alizarin Red S (Sigma)), washed three times with distilled water to remove unbound staining and air-dried. Quantification of the staining was performed by dissolving the mineral content in 800 µl of 10% acetic acid for 30 minutes. Then, the wells were scraped, the content of each well was harvested in a separate tube and vortexed for 30 seconds. Each tube was heated at 85°C for 10 minutes, then put on ice for 5 minutes. Finally, tubes were centriguated at 13000 rpm for 15 minutes, the liquid phase was harvested and mixed with 100 µl of 10% ammonium hydroxide. Absorbance was measured at 405 nm with a spectrophotometer (Varioskan Flash, Thermo Fisher Scientific).

### Protein extraction and Western blot analysis (to see activation of canonical and non-canonical Wnt pathways)

Proteins were isolated from the micromasses using 1x Laemmli Buffer (2%SDS, 10% glycerol, 5% 2-betamercaptoethanol, 0.002% bromophenol blue, 125 mM Tris HCI (pH 6.8)). Samples were denatured for 5 min at 95°C, chilled on ice and separated on a Tris-Glycine extended gradient gel 4-20% (Biorad) by electrophoresis using a migration buffer, containing 192 mM glycine, 20 mM Tris Base, 0,1% SDS. (Invitrogen). Then, proteins were transferred onto a polyvinylidine difluoride (PVDF) membrane, using the Trans-blot turbo system (BioRad) during 7 minutes. After 2 h in blocking buffer (TBS-0.1% Tween (TBST) supplemented with 5% non-fat dry milk) at room temperature, membranes were washed three times in TBST and incubated overnight at 4°C with one primary antibody against either active β-catenin, total β-catenin, phospho CamKII or total CamKII in TBST-5% bovine serum albumine. After three washes with TBST, each blot was incubated for 1 h at room temperature with anti-Rabbit or anti-mouse antibodies conjugated with horseradish peroxidase (Jackson ImmunoResearch Laboratories) in blocking buffer. Blots were visualized using Clarity Western ECL substrate (BioRad) according to manufacturer's recommendations. Images were acquired with the ChemiDoc XRS CCD camera system (BioRad). Densitometry analysis was performed with ImageJ Software (NIH Image, National Institutes of Health, Bethesda, MD, USA). Results are expressed as the ratio of active (phospho) protein form/total protein form.

### MMP13 enzymatic activity assay

The MMP13 enzymatic activity was assessed using the MMP13 fluorogenic substrate (Merck Millipore). Briefly, culture supernatants harvested at the end of the different experiments were mixed with 1.5 mM of 4-aminophenylmercuric acetate, and incubated for 1 h at 37°C, in order to turn every pro-MMPs into active MMPs. Then, the fluorogenic substrate was added, and the mixture was incubated for 3 h at 37°C. Fluorescence was evaluated using the following parameters, excitation 325 nm and emission 393 nm, on the Varioskan Flash device (Thermo Fisher Scientific). Recombinant human MMP13 was used as a positive control (Merck Millipore). Negative control consisted in growth medium.

### Statistical analyses

Statistical analyses were carried out using the GraphPrism v 6.0. ANOVA were performed to assess the differences between control groups (cells in absence of Wnt3a, IL1β or PEP16) and treated groups (Wnt3a alone, IL1β alone, PEP16 alone or PEP16bis (SEQ ID NO: 3) alone), and also between Wnt3a treated groups and Wnt3a + PEP16, Wnt3a + PEP16bis groups.

### EXAMPLE 2 : RESULTS

### Treatment of the ATDC5 cell line with the PEP16 at 30, 100, 300 ng/mL (respectively. P30, P100 and P300)

Results of relative expression level of (A) *aggrecan,* (B) *col2a1,* (C) *ctgf,* (D) *mmp3* and (E) *mmp13,* at day 1, 7, 14, 21 and 28 in ATDC5 micromasses treated with the PEP16 at 30, 100, 300 ng/mL, are shown in Figure 2.

The results of glycosaminoglycans quantification (A) with computer program (blue pixels quantification) (B) with biochemical quantification (solubilisation of the staining in Guanidine HCl 6M), in ATDC5 micromasses treated with the PEP16 at 30, 100 and 300 ng/mL (respectively, P30, P100 and P300) and cultured during 28 days, are shown in Figure 6.

The results of western blot exploring (A) the Wnt/β-catenin pathway activation (B) the Camkll pathway activation, in ATDC5 micromasses at day 1, 7, 14, 21 and 28 after treatment with the PEP16 at 100 and 300 ng/mL are shown in Figure 7. 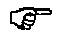 The results obtained show that PEP16 significantly enhanced the induction of the expression of markers of the healthy joint cartilage phenotype during ATDC5 chondrogenesis (aggrecan and type 2 collagen). Moreover, PEP16 efficiently reduced the expression of osteoarthritis-related MMPs, i.e. MMP3 and MMP13, as well as the expression of Ctgf, a target genes of the Hippo/YAP/TAZ pathway. In addition, PEP16 significantly increased the accumulation of glycosaminoglycans in the extracellular matrix during chondrogenesis. Finally, PEP16 increased the activation of the CamKII pathway in the pre-mineralization phase of chondrogenesis (D14), while it reduced in the activation of the Wnt/β-catenin pathway during chondrogenesis.

### Treatment of the ATDC5 cell line with the PEP16 and PEP16bis at 300ng/mL

The results of relative expression level of (A) *aggrecan,* (B) *col2a1,* (C) *mmp3,* (D) *mmp13,* and (E) *ankrd* at day 1, 21 and 28 in ATDC5 micromasses treated with the PEP16 or PEP16bis at 300 ng/mL, are shown in Figure 3.

The results of glycosaminoglycans quantification at day 28 in ATDC5 micromasses treated with the PEP16 or the PEP16bis at 300 ng/mL are shown in Figure 4.

The results of promoter activation assays in ATDC5 cells transfected with constructs containing the firefly luciferase gene under the control of (A) 7X TCF/LEF response elements (Super 8x TOPFlash reporter) in ATDC5 micromasses treated one day after transfection with the PEP16 or the PEP16bis at 300 ng/mL and the Wnt3a at 10 ng/mL for 24h, (B) 8X YAP reponse elements (8x GTIIC reporter) or (C) 7X TCF/LEF response elements (Super 8x TOPFlash reporter) in ATDC5 micromasses treated one day after with the PEP16 PEP16 at 3, 30, 100, 300 ng/mL (respectively P3, P30, P100 and P300) and the Wnt3a at 100 ng/mL for 24h, are shown in Figure 5.

The results of western blot exploring (A) the Wnt/β-catenin pathway activation (B) the Camkll pathway activation, in ATDC5 micromasses at day 21 and 28 after treatment with the PEP16 or the PEP16bis at 300 ng/mL are shown in Figure 8. 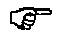 The results obtained show that PEP16bis also favoured the induction of the expression of markers of the healthy joint cartilage phenotype during ATDC5 chondrogenesis (aggrecan and type 2 collagen). Moreover, PEP16bis efficiently reduced the expression of osteoarthritis-related MMPs, i.e. MMP3 and MMP13, as well as the expression of Ctgf, a target gene of the Hippo/YAP/TAZ pathway. In addition, PEP16bis significantly increased the accumulation of glycosaminoglycans in the extracellular matrix during chondrogenesis. Furthermore, PEP16 and PEP16bis at 300 ng/ml were able to significantly reduce the activity of the canonical pathway in the Super TOPFlash luciferase reporter system (ATDC5 cells transfected with the reporter plasmid, and stimulated with 10 or 100 ng/mL of recombinant Wnt3a protein which activates the system), while the other four sequences are unable to do so. Similarly, PEP16 at 30 ng/ml significantly reduced the activity of the Hippo/YAP/TAZ pathway in a luciferase-type reporter system (ATDC5 cells transfected with the reporter plasmid of the YAP pathway, which activates spontaneously on contact with the plastic). Finally, PEP16bis increased the activation of the CamKII pathway, while it reduced in the activation of the Wnt/β-catenin pathway during chondrogenesis.

### Treatment of mouse articular chondrocytes culture with PEP16 or PEP16bis at 300ng/mL

The results of MMP13 enzymatic activity in culture supernatants of mouse articular chondrocytes treated during 24h with PEP16 at 300 ng/mL and/or with IL1β at 100 pg/mL as a positive control for MMP13 activity, are shown in Figure 9.

The results of the relative expression level of (A) *aggrecan,* (B) *col2a1,* at 24 and 48h in mouse articular chondrocytes treated with the PEP16 at 300 ng/mL and/or wnt3a at 100 ng/mL, are shown in Figure 10.

The results of the relative expression level of (A, B) *mmp3* and (C) ankrd, in mouse articular chondrocytes treated with the PEP16 or PEP16bis at 300 ng/mL, and/or wnt3a at 100, 50, 30 ng/mL, are shown in Figure 11. 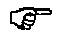 The results obtained show a protective role of the peptides under test. PEP16 significantly reduced the IL1β-induced MMP13 activity measured in the culture supernatant of mouse articular chondrocytes. Moreover, PEP16 had a tendancy to counteract the deleterious effects of β-catenin activation by Wnt3a, on the decrease of markers of the healthy joint cartilage phenotype (aggrecan and type 2 collagen), and on the increase of NGF (pain, data not shown). The peptides under test are also able to counteract the deleterious effects induced by the use of the recombinant Wnt3a, in particular, PEP16 counteract the inductive effects of Wnt3a on the expression of MMP3. In addition, PEP16 and PEP16bis are capable of reducing the expression of Ankrd, a target gene of the Hippo/YAP/TAZ pathway in this primary culture system. A similar trend is observed for Cyr61 and Ctgf, two other target genes of the Hippo/YAP/TAZ pathway.

### Treatment of diseased human articular cartilage explants (osteoarthritis) with the PEP16 at 300 ng/mL

The results of the MMP13 enzymatic activity in culture supernatants of human articular cartilage explants (from 3 patients with end-stage osteoarthritis following total knee replacement surgery) treated with PEP16 at 300 ng/mL for 7 days (3 stimulations in 7 days), are shown in Figure 12. 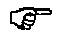 The results obtained show that PEP16 is capable of reducing the enzymatic activity of MMP13 measured in the culture supernatant of human cartilage explants.

### Treatment of MC3T3-E1 cell line with the PEP16

The results of relative expression level of *osteocalcin* and *osterix* at day 7, 14 and 21 in MC3T3-E1 cells treated with the PEP16 at 100 ng/mL or 300 ng/mL (P100 and P300, respectively), are shown in Figure 13A. The results of western blot exploring the Wnt/β-catenin pathway and the Camkll pathway activation, in MC3T3-E1 cells at day 7, 14 and 21 after treatment with the PEP16 at 300 ng/mL or 1000 ng/mL (P300 and P1000 respectively) are shown in Figure 13B. The results of mineralization quantification at day 21 in MC3T3-E1 cells treated with the PEP16 at 100 ng/mL or 300 ng/mL are shown in Figure 13C. 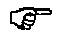 The results obtained show that PEP16 favoured the induction of the expression of osteogenic markers during MC3T3-E1 osteogenic differentiation (osteocalcin and osterix). Moreover, PEP16 efficiently increased the activation of the CamKII pathway in the early osteogenesis stages, while it reduced in the activation of the Wnt/β-catenin pathway during osteogenesis. In addition, PEP16 significantly increased the accumulation of mineral content in the extracellular matrix. These results are in favour of using PEP16 in the prevention or treatment of osteoporosis, although it is quite counter-intuitive. Indeed, in this disease, attempts are usually made to promote the Wnt/β-catenin pathway, notably with anti-sclerostin antibodies.

### List of references

1. Lories and Luyten, Nat Rev Rheumatol, 7(1): p. 43-9, 2011
2. Hunter and Felson, BMJ,_332(7542): p. 639-42, 2006
3. Valdes and Spector, Rheum Dis Clin North Am, 34(3): p. 581-603, 2008
4. Lories et al., Arthritis Rheum, 56(12): p .4095-103, 2007
5. Lodewyckx et al., Arthritis Res Ther, 14(1): R16, 2012
6. Lane et al., Arthritis Rheum, 56(10): p. 3319-25, 2007
7. Rockel et al., J Clin Invest, 126(5): p. 1649-63, 2016
8. Zhu et al., Arthritis Rheum, 58(7): p. 2053-2064, 2008
9. Tamamura et al., J Biol Chem, 280(19): p. 19185-95, 2005
10. Zhu et al., J Bone Miner Res, 24(1): p. 12-21, 2009
11. Gong et al, J Mol Med, 97(1):103-114. doi: 10.1007/s00109-018-1705-y, 2019
12. Zhang et al, Biomaterials, 232:119724. doi:10.1016/j.biomaterials.2019.119724, 2020
13. Li et al., Development, 2011; 138(2): p. 359-70.
14. Nalesso et al., Osteoarthritis Cartilage, 24 Suppl 1: S149, 2016
15. Thysen et al., Lab Invest, 96(5): p. 570-80, 2016
16. Thysen et al., Ann Rheum Dis, 73 Suppl 1:A67-8, 2014
17. Cui et al., Trends Biochem Sci, 43(8): p. 623-634, 2018
18. Zheng and Pan, Dev Cell, 50(3): p. 264-282, 2019

## Claims

1. Peptide of 16 to 50 amino acids comprising a peptide having the following sequence :
DRX₃GKKVKRWDMKX₁₄RH (SEQ ID NO: 1)
wherein X₃ and X₁₄ are independently of each other any amino acid.

2. Peptide according to claim 1, wherein X₃ and X₁₄ are independently of each other an alanine or a leucine.

3. Peptide according to claim 1 or 2 comprising the sequence SEQ ID NO: 2 (DRLGKKVKRWDMKLRH) or SEQ ID NO: 3 (DRAGKKVKRWDMKARH).

4. Peptide according to any one of claims 1 to 3, for use as a drug.

5. Pharmaceutical composition comprising a peptide according to any one of claim 1 to 3, and one or more pharmaceutically acceptable excipient.

6. Peptide according to any one of claim 1 to 3 or pharmaceutical composition according to claim 5, for use in the prevention or treatment of a Wnt/β-catenin and/or Hippo/YAP/TAZ pathway-related disease selected from the group consisting of: osteoarthritis, osteoporosis, cancer.

7. Peptide or pharmaceutical composition for use according to claim 6, in the prevention or treatment of osteoarthritis.


SEQUENCE LISTING

<110>  Université de Lorraine
       Centre National de la Recherche Scientifique (CNRS)
       CAILOTTO, Frédéric
       CLAUDEL, Marion
       CHARRON, Christophe
       JOUZEAU, Jean-Yves

<120>  THERAPEUTIC POTENTIAL OF PEPTIDES FROM THE "NETRIN-LIKE" DOMAIN
       OF THE FRZB PROTEIN

<130>  BNT226529EP00

<160>  8

<170>  PatentIn version 3.5

<210>  1
<211>  16
<212>  PRT
<213>  Artificial Sequence

<220>
<223>  Sequence consensus PEP


<220>
<221>  MISC_FEATURE
<222>  (3)..(3)
<223>  Xaa is any amino acid

<220>
<221>  MISC_FEATURE
<222>  (14)..(14)
<223>  Xaa is any amino acid

<400>  1

Asp Arg Xaa Gly Lys Lys Val Lys Arg Trp Asp Met Lys Xaa Arg His
1               5                   10                  15


<210>  2
<211>  16
<212>  PRT
<213>  Artificial Sequence

<220>
<223>  PEP16

<400>  2

Asp Arg Leu Gly Lys Lys Val Lys Arg Trp Asp Met Lys Leu Arg His
1               5                   10                  15


<210>  3
<211>  16
<212>  PRT
<213>  Artificial Sequence

<220>

<223>  PEP16bis

<400>  3

Asp Arg Ala Gly Lys Lys Val Lys Arg Trp Asp Met Lys Ala Arg His
1               5                   10                  15


<210>  4
<211>  12
<212>  PRT
<213>  Artificial Sequence

<220>
<223>  PEP12

<400>  4

Ala Thr Gln Lys Thr Tyr Phe Arg Asn Asn Tyr Asn
1               5                   10


<210>  5
<211>  16
<212>  PRT
<213>  Artificial Sequence

<220>
<223>  PEP16 mutant

<400>  5

Asp Arg Leu Gly Lys Lys Val Lys Ala Trp Asp Met Lys Leu Arg His
1               5                   10                  15


<210>  6
<211>  16
<212>  PRT
<213>  Artificial Sequence

<220>
<223>  PEP16 mutant

<400>  6

Asp Lys Leu Gly Arg Arg Val Arg Lys Trp Asp Met Arg Leu Lys His
1               5                   10                  15


<210>  7
<211>  16
<212>  PRT
<213>  Artificial Sequence

<220>
<223>  PEP16 mutant

<400>  7

Asp Arg Ala Gly Lys Lys Ala Lys Arg Trp Asp Met Lys Ala Arg His
1               5                   10                  15

<210>  8
<211>  16
<212>  PRT
<213>  Artificial Sequence

<220>
<223>  PEP16 mutant

<400>  8

Asp Arg Leu Gly Lys Lys Val Lys Arg Ala Asp Met Lys Leu Arg His
1               5                   10                  15